# EUROPEAN PATENT APPLICATION

(11) **EP 4 635 474 A1**
(43) Date of publication of application: **22.10.2025**
(21) Application number: 23903700.5
(22) Date of filing: 10.10.2023
(51) Int. Cl.: A61K 8/98, A61K 35/545, A61P 17/00, A61Q 19/00, A61Q 19/08, C12N 5/0775

(54) **COMPOSITION FOR INHIBITING SKIN AGING COMPRISING STEM CELL-DERIVED EXOSOMES AND METHOD FOR PRODUCING SAME**

(30) Priority: 16.12.2022 KR 20220177466
(71) Applicant: Brexogen Inc., Seoul 05855 (KR)
(72) Inventor: KIM, Sue, Seoul 05855 (KR); JUNG, Minyoung, Seoul 05803 (KR)
(74) Representative: Plougmann Vingtoft a/s
(86) International application number: PCT/KR2023/015500
(87) International publication number: WO 2024/128496

(57) **Abstract**

The present invention relates to: a composition for inhibiting skin aging, the composition comprising stem cell-derived exosomes; and a method for producing same. The composition comprising exosomes according to the present invention is highly effective in preventing skin stretch marks, improving skin elasticity, preventing skin aging, or promoting skin regeneration, and thus can be used in various cosmetic and pharmaceutical compositions.

## Description

### Technical Field

The present disclosure relates to a composition for inhibiting skin aging comprising stem cell-derived exosomes and a method for preparing same and, more particularly, to a composition comprising exosomes isolated from mesenchymal stem cells or a culture thereof, which exhibit excellent effects in preventing stretch marks, enhancing skin elasticity, preventing skin aging, or promoting skin regeneration.

### Background Art

Extracellular vesicles include microvesicles and exosomes and are vesicles composed of a spherical lipid bilayer structure having a size of 30 to 1000 nm.

The lipid bilayer of an exosome has a phospholipid double membrane structure similar to that of the originating cell (donor cell), and exosomes are known to function as structures secreted by cells to the extracellular space, playing functional roles in intercellular communication and immunomodulation.

Exosomes contain cell-specific components that reflect the biological characteristics of the originating cell, and include various constituents such as phospholipids, mRNA, miRNA, soluble proteins, surface proteins, and transmembrane proteins.

Such exosomes are secreted from various animal cells, including mast cells, lymphocytes, astrocytes, platelets, neurons, endothelial cells, and epithelial cells, and are found in various body fluids such as blood, urine, mucus, saliva, bile, ascitic fluid, and cerebrospinal fluid. Exosomes are also capable of crossing the blood-brain barrier (BBB) and are highly selectively permeable to the extent that they can pass through the membranes of epidermal and endothelial cells, and thus have been used in the development of drug delivery systems (DDS) as nanocarriers for specific drugs.

Exosomes and microvesicles secreted from mesenchymal stem cells are involved in cell-to-cell communication and are known to exhibit therapeutic efficacy in regenerative medicine attributed to stem cells.

It is known that when stem cells are transplanted into the body, even without long-term survival, paracrine factors secreted from the cells can exert trophic effects. These factors include low-molecular-weight substances such as growth factors, chemokines, and cytokines, which are secreted via extracellular vesicles such as exosomes, and such exosomes are derived from stem cells. Therefore, exosomes are utilized in identifying the characteristics of stem cells and evaluating their therapeutic efficacy. Furthermore, recent research has actively focused on using exosomes secreted by mesenchymal stem cells, rather than using the mesenchymal stem cells themselves, for the treatment of various diseases. It is expected in both academia and industry that such an approach can provide a new alternative to overcome the limitations of conventional stem cell therapies.

### Disclosure of Invention

### Technical Problem

Research conducted by the present inventors resulted in developing a composition isolated from stem cells or a culture thereof, and finding that the composition according to the present disclosure exhibits significantly superior effects in preventing stretch marks, improving skin condition, particularly enhancing skin elasticity, preventing skin aging, and promoting skin regeneration.

Accordingly, an aspect of the present disclosure is to provide a composition including stem cell-derived exosomes.

Another aspect of the present disclosure is to provide a method for preparing a composition including stem cell-derived exosomes.

Yet another aspect of the present disclosure is to provide a composition for preventing stretch marks, enhancing skin elasticity, preventing skin aging, or promoting skin regeneration, the composition including stem cell-derived exosomes.

Still another aspect of the present disclosure is to provide a method for preventing stretch marks, enhancing skin elasticity, preventing skin aging, or promoting skin regeneration using stem cell-derived exosomes.

A further aspect of the present disclosure is to provide the use of a composition including stem cell-derived exosomes in preventing stretch marks, enhancing skin elasticity, preventing skin aging, or promoting skin regeneration.

### Solution to Problem

In view of the above, the present inventors have confirmed that the composition according to the present disclosure exhibits excellent effects in preventing stretch marks, enhancing skin elasticity, preventing skin aging, or promoting skin regeneration.

Hereinafter, a detailed description will begiven of the present disclosure.

One aspect of the present disclosure relates to a composition comprising exosomes derived from stem cells.

As used herein, the term "exosome" refers to a cell-derived vesicle that exists in the body fluids of almost all eukaryotic organisms. It is a vesicle with a diameter of approximately 30-100 nm, which is larger than low-density lipoprotein (LDL) proteins but much smaller than red blood cells. Exosomes may be released when multivesicular bodies (MVBs) fuse with the plasma membrane or may be directly secreted from the plasma membrane, and they are known to perform important and specialized functions such as coagulation and intercellular signaling.

As used herein, the term "stem cell" refers to an undifferentiated cell that has the ability to self-renew and differentiate into two or more different types of cells.

In an embodiment of the present disclosure, the stem cell may be an autologous or allogeneic stem cell, may be derived from any type of animal including humans and non-human mammals, and may be an adult-derived or embryonic-derived stem cell. For example, the stem cell may be selected from the group consisting of embryonic stem cells, adult stem cells, induced pluripotent stem cells (iPSCs), mesenchymal stem cells derived from iPSCs, BxC stem cells, mesenchymal stem cells derived from iPSCs pretreated with hyaluronic acid (HA), and BxC-R11 stem cells, but is not limited thereto.

As used herein, the term "adult stem cell" refers to a cell extracted from umbilical cord blood, adult bone marrow, or blood, which is a cell in a state just before differentiation into cells of specific organs, and refers to an undifferentiated cell having the potential to develop into tissue within the body when necessary.

In an embodiment of the present disclosure, the adult stem cell may be selected from the group consisting of adult stem cells derived from humans, animals, or animal tissues; mesenchymal stromal cells derived from humans, animals, or animal tissues; and induced pluripotent stem cell-derived mesenchymal stem cells of human or animal origin, but is not limited thereto.

In the present disclosure, the human or animal tissue may be selected from the group consisting of umbilical cord, umbilical cord blood, bone marrow, adipose tissue, muscle, nerve, skin, amnion, and placenta, but is not limited thereto.

In the present disclosure, the stem cell derived from various human or animal tissues may be selected from the group consisting of hematopoietic stem cells, mammary stem cells, intestinal stem cells, vascular endothelial stem cells, neural stem cells, olfactory neural stem cells, and testicular stem cells, but is not limited thereto.

As used herein, the term "embryonic stem cell" refers to a cell extracted during the developmental stage of an embryo, and more specifically, refers to a culture obtained by isolating the inner cell mass from a blastocyst-stage embryo just before implantation into the uterus and culturing it in vitro.

Embryonic stem cells are pluripotent or totipotent cells with self-renewal ability, which can differentiate into all types of tissues in an organism. In a broad sense, the term also encompasses embryoid bodies derived from embryonic stem cells.

The stem cell used in the present disclosure may include embryonic stem cells derived from all species, including humans, monkeys, pigs, horses, cattle, sheep, dogs, cats, mice, and rabbits, but is not limited thereto.

As used herein, the term "induced pluripotent stem cell (iPSC)" refers to a cell that has been artificially reprogrammed from a differentiated cell to possess pluripotency, and may be used interchangeably with the term "dedifferentiated stem cell."

The artificial reprogramming process may be carried out by introducing reprogramming factors via virus-mediated methods using retroviruses, lentiviruses, or Sendai viruses, or by non-viral methods such as non-viral vectors, proteins, and cell extracts. It may also include reprogramming by stem cell extracts or chemical compounds.

Induced pluripotent stem cells (iPSCs) possess nearly identical characteristics to embryonic stem cells. Specifically, they exhibit similar cell morphology, gene and protein expression patterns, have pluripotency both *in vitro* and *in vivo,* form teratomas, and are capable of forming chimera mice and undergoing germline transmission when injected into the blastocyst of a mouse.

In an embodiment of the present disclosure, the stem cell may be an induced pluripotent stem cell-derived mesenchymal stem cell.

In an embodiment of the present disclosure, the composition may comprise exosomes derived from induced pluripotent stem cell-derived mesenchymal stem cells.

As used herein, the term "mesenchymal stem cell" (MSC) refers to a stem cell derived from the mesenchyme. Mesenchymal stem cells can differentiate into one or more cell types selected from the group consisting of osteoblasts, chondrocytes, adipocytes, and myocytes. Mesenchymal stem cells may be isolated from all types of adult tissues, for example, from bone marrow, adipose tissue, umbilical cord, or peripheral blood. A population of mesenchymal stem cells may be defined by a specific phenotype. A population of induced pluripotent stem cell-derived mesenchymal stem cells may exhibit the same phenotypic characteristics as a conventional mesenchymal stem cell population. For example, mesenchymal stem cell populations may be understood to express CD105, CD73, and CD90 markers at levels of 95% or higher, and to express CD45, CD34, and SSEA-4 at levels of 2% or lower.

In an embodiment of the present disclosure, the stem cell may be a BxC stem cell.

In an embodiment of the present disclosure, the composition may comprise exosomes derived from BxC stem cells.

As used herein, the term "BxC stem cell" refers to a stem cell obtained by culturing induced pluripotent stem cells (iPSCs), isolating a population of iPSCs that do not express stage-specific embryonic antigen 4 (SSEA-4), and further culturing the isolated cells. BxC stem cells are at a stage just before complete differentiation from iPSCs into mesenchymal stem cells, and may acquire full mesenchymal stem cell characteristics through additional culturing. Therefore, the phenotype of a BxC stem cell population is not completely identical to that of a mesenchymal stem cell population but may be similar within a range of 96% to 99.9%. For example, a population of iPSCs may express CD90 at a level of 0.3%, while a population of mesenchymal stem cells expresses CD90 at 99.7%, and a population of BxC stem cells may express CD90 at approximately 96.9%, which is about 98% of the level observed in mesenchymal stem cells. Accordingly, BxC stem cells may be defined as stem cells that are cultured from iPSCs lacking SSEA-4 expression and differentiated into mesenchymal stem cells at a level of 96% to 99.9% without complete differentiation. BxC stem cells exhibit superior stemness compared to mesenchymal stem cells derived from iPSCs, and are capable of secreting large amounts of functionally relevant proteins. Specifically, BxC stem cells according to the present disclosure exhibit more than a 10-fold difference in proliferation capacity compared to mesenchymal stem cells derived from the same tissue origin after 9 or more passages, and do not show reduced proliferation even after 12 or more passages. In addition, BxC stem cells exhibit more than twice the expression level of Ki67, a marker associated with cell proliferation, compared to general mesenchymal stem cells. Furthermore, BxC stem cells may express one or more genes at higher levels selected from the group consisting of ANKRD1, CPE, NKAIN4, LCP1, CCDC3, MAMDC2, CLSTN2, SFTA1P, EPB41L3, PDE1C, EMILIN2, SULT1C4, TRIM58, DENND2A, CADM4, AIF1L, NTM, SHISA2, RASSF4, and ACKR3, and may express one or more genes at lower levels selected from the group consisting of DHRS3, BMPER, IFI6, PRSS12, RDH10, and KCNE4.

As used herein, the term "stemness" refers to the ability of a stem cell to generate all types of cells (pluripotency) and the ability to indefinitely self-renew. For example, stemness may be represented by one or more of the following: maintaining the undifferentiated state while increasing the proliferation capacity of stem cells, increasing telomerase activity, increasing the expression of stemness-acting signals, or increasing cell motility.

In an embodiment of the present disclosure, the stem cell may be an induced pluripotent stem cell-derived mesenchymal stem cell that has been pretreated with hyaluronic acid (HA).

In an embodiment of the present disclosure, the composition may comprise exosomes derived from induced pluripotent stem cell-derived mesenchymal stem cells pretreated with hyaluronic acid (HA).

As used herein, the term "pretreatment" refers to a process of culturing mesenchymal stem cells in a medium supplemented with a specific substance. For example, pretreatment may refer to additionally culturing mesenchymal stem cells, which have been fully differentiated from iPSCs, in a medium containing hyaluronic acid (HA).

In the present disclosure, hyaluronic acid may enhance the stemness and proliferation of stem cells and may increase the number of stem cell-derived exosomes, as well as the protein and RNA content within the exosomes.

In an embodiment of the present disclosure, the stem cell may be a BxC-R11 stem cell.

In an embodiment of the present disclosure, the composition may include exosomes derived from BxC-R11 stem cells (BxC-R11e).

As used herein, the term "BxC-R11 stem cell" refers to a mesenchymal stem cell obtained by fully differentiating a BxC stem cell into a mesenchymal stem cell and then culturing the resulting cell in a medium containing hyaluronic acid (HA) (i.e., pretreatment). For example, BxC-R11 stem cells may be prepared by further culturing BxC stem cells to fully differentiate them into mesenchymal stem cells, and then culturing them for 12 to 48 hours in a medium containing 0.1 to 1000 µg/mL, for example, 40 µg/mL of hyaluronic acid. BxC-R11 stem cells exhibit approximately 360% higher proliferation rates compared to untreated mesenchymal stem cells, a fivefold increase in exosome production efficiency, and more than a fivefold increase in the amount of protein derived from the exosomes.

In an embodiment of the present disclosure, hyaluronic acid may be used for pretreatment at a concentration of 0.1 to 1000 µg/mL, 0.5 to 1000 µg/mL, 1 to 500 µg/mL, 1 to 200 µg/mL, 1 to 100 µg/mL, 1 to 80 µg/mL, 1 to 60 µg/mL, or 10 to 60 µg/mL, and may, for example, be used at 40 µg/mL, but is not limited thereto.

Another aspect of the present disclosure provides a cosmetic composition including exosomes isolated from induced pluripotent stem cell (iPSC)-derived mesenchymal stem cells as an active ingredient for preventing stretch marks, enhancing skin elasticity, preventing skin aging, or promoting skin regeneration.

Another aspect of the present disclosure provides a cosmetic composition including exosomes isolated from induced pluripotent stem cell (iPSC)-derived mesenchymal stem cells, as an active ingredient for enhancing skin elasticity.

Another aspect of the present disclosure provides a cosmetic composition including exosomes isolated from induced pluripotent stem cell (iPSC)-derived mesenchymal stem cells as an active ingredient for preventing skin aging.

Another aspect of the present disclosure provides a cosmetic composition including exosomes isolated from induced pluripotent stem cell (iPSC)-derived mesenchymal stem cells as an active ingredient for promoting skin regeneration.

Another aspect of the present disclosure provides a cosmetic composition including exosomes isolated from induced pluripotent stem cell (iPSC)-derived mesenchymal stem cells as an active ingredient for preventing stretch marks.

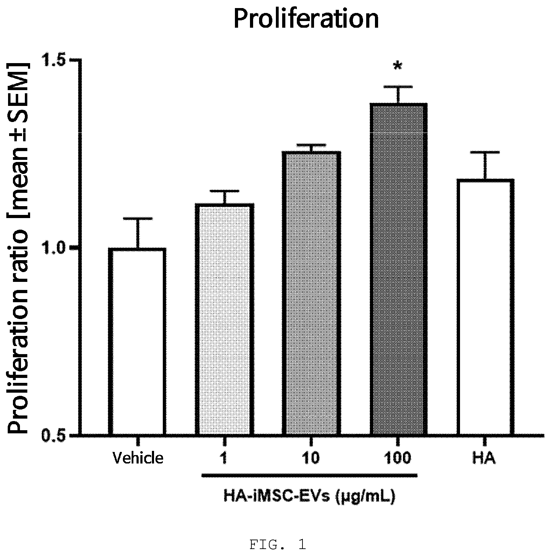

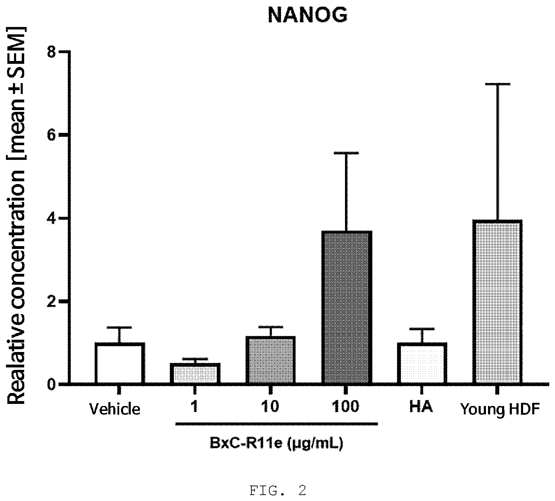

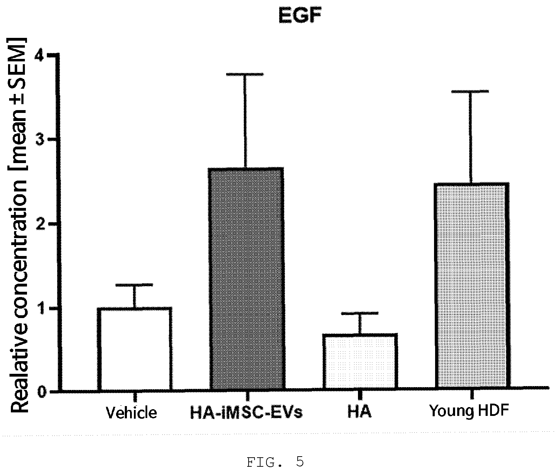

As used herein, the term "regeneration" refers to the process of recovery of skin tissue from skin (cellular) damage caused by external or internal factors. External causes of such damage may include ultraviolet rays, environmental pollutants, wounds, or trauma, and internal causes may include stress, among others. The composition according to the present disclosure significantly improves cell proliferation when brought into contact not only with normal or young dermal fibroblasts but also with aged human dermal fibroblasts (HDFs) (see Example 3 and FIG. 1), promotes the expression of the NANOG gene, which is closely associated with cellular regeneration (see Example 4 and FIG. 2), and enhances the expression of epidermal growth factor (EGF) and elastin genes in dermal fibroblasts (see Example 6 and FIG. 5), thereby exhibiting outstanding effects in promoting skin regeneration and being suitable for such applications.

As used herein, the term "skin elasticity" refers to the skin's ability to return to its original shape, which is governed by elastin and collagen present in the dermis layer. When dermal fibroblasts are activated, the maintenance of elastin and collagen content enhances skin elasticity. Therefore, the composition comprising exosomes according to the present disclosure significantly improves cell proliferation when brought into contact with aged fibroblasts of animals including humans (see Example 3 and FIG. 1), promotes the expression of epidermal growth factor (EGF) and elastin genes in fibroblasts (see Example 6 and FIG. 5), and not only enhances elastin protein expression but also promotes the assembly of elastin fibers, thus confirming its excellent efficacy in improving skin elasticity.

As used herein, the term "skin aging" refers to symptoms such as reduced elasticity, loss of luster, formation of wrinkles, weakened regenerative ability, or severe dryness in the skin, which may be caused by the passage of time or external environmental factors. Accordingly, the composition comprising exosomes according to the present disclosure demonstrates excellent efficacy in promoting fibroblast proliferation (see Examples 3 and 4 and FIG. 1), reducing SA-β-Gal activity (see Example 5 and FIG. 4), and promoting the expression of epidermal growth factor and elastin genes (see Example 6 and FIG. 5), and thus may be used for the prevention or improvement of skin aging.

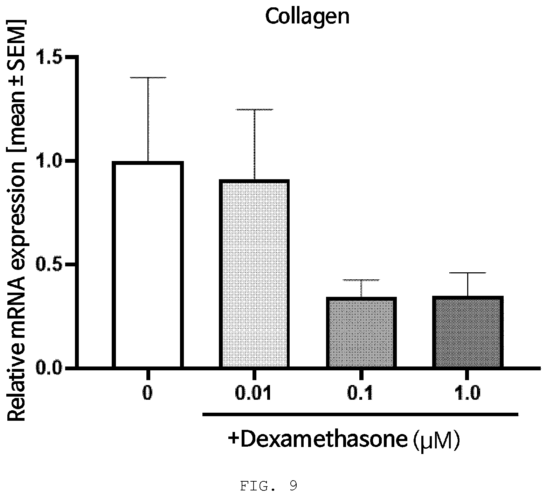

As used herein, the term "prevention of stretch marks" refers to the prevention of striae distensae, which are medically defined as atrophic linear bands that form due to the stretching of the skin, resulting in loosening or breaking of collagen fibers. In the skin lesions of patients with striae distensae, collagen and elastin levels are known to be reduced, and collagen-elastin hydrolysates have been reported to be effective in preventing stretch marks, indicating that collagen and elastin play a key role. The composition comprising exosomes according to the present disclosure has been confirmed to promote the expression of the elastin gene (see Example 6 and FIG. 5), enhance elastin protein expression, and promote the assembly of elastin fibers, thereby exhibiting excellent efficacy in preventing stretch marks. Notably, the composition of the present disclosure significantly promotes the expression of collagen and elastin genes even when skin cells are treated with dexamethasone, which suppresses the expression of collagen and fibroblasts, and does so more effectively than hyaluronic acid (see Example 8 and FIGS. 9 to 10).

As used herein, the expression "including as an active ingredient" means that the composition includes an amount of exosomes isolated from stem cells or a culture thereof sufficient to exert a particular effect, such as the prevention of stretch marks, enhancement of skin elasticity, prevention of skin aging, or promotion of skin regeneration.

In an embodiment of the present disclosure, the mesenchymal stem cell derived from an induced pluripotent stem cell may be differentiated from a precursor cell of the mesenchymal stem cell that does not express the stage-specific embryonic antigen 4 (SSEA-4) protein.

In an embodiment of the present disclosure, the cosmetic composition may be in a formulation selected from the group consisting of solution, topical ointment, cream, foam, nourishing toner, softening toner, pack, softener, emulsion, makeup base, essence, soap, liquid cleanser, bath preparation, sunscreen cream, sun oil, suspension, emulsion, paste, gel, lotion, powder, soap, surfactant-containing cleanser, oil, powder foundation, emulsion foundation, wax foundation, patch, and spray, but is not limited thereto.

When the formulation of the present disclosure is an ointment, paste, cream, or gel, the carrier component may include, but is not limited to, animal oil, vegetable oil, wax, paraffin, starch, tragacanth, cellulose derivatives, polyethylene glycol, silicone, bentonite, silica, talc, or zinc oxide. These may be used individually or in combination of two or more.

When the formulation of the present disclosure is a powder or spray, the carrier component may include, but is not limited to, lactose, talc, silica, aluminum hydroxide, calcium silicate, or polyamide powder. Particularly, in the case of a spray, a propellant such as chlorofluorohydrocarbon, propane/butane, or dimethyl ether may additionally be included. These may be used individually or in combination of two or more.

When the formulation of the present disclosure is a solution or emulsion, the carrier component may include a solvent, solubilizer, or emulsifier. Examples include, but are not limited to, water, ethanol, isopropanol, ethyl carbonate, ethyl acetate, benzyl alcohol, benzyl benzoate, propylene glycol, 1,3-butylene glycol, and oils. In particular, oils such as cottonseed oil, peanut oil, corn germ oil, olive oil, castor oil, sesame oil, glycerol fatty acid esters, polyethylene glycol, or fatty acid esters of sorbitan may be used. These may be used individually or in combination of two or more.

When the formulation of the present disclosure is a suspension, the carrier component may include a liquid diluent such as water, ethanol, or propylene glycol; a suspending agent such as ethoxylated isostearyl alcohol, polyoxyethylene sorbitol ester, or polyoxyethylene sorbitan ester; and optionally microcrystalline cellulose, aluminum metahydroxide, bentonite, agar, or tragacanth, but is not limited thereto. These may be used individually or in combination of two or more.

Another aspect of the present disclosure provides a pharmaceutical composition for preventing stretch marks, enhancing skin elasticity, preventing skin aging, or promoting skin regeneration, comprising, as an active ingredient, exosomes isolated from induced pluripotent stem cell (iPSC)-derived mesenchymal stem cells.

The pharmaceutical composition of the present disclosure may further comprise a pharmaceutically acceptable carrier.

As used herein, the term "pharmaceutically acceptable" refers to a substance that does not cause irritation to the subject upon administration and does not adversely affect the biological activity and properties of the administered compound, and is commonly used in the field of pharmaceuticals.

In the present disclosure, any carrier commonly used in the art may be employed. Non-limiting examples of carriers include saline, sterile water, Ringer's solution, buffered saline, albumin injection solution, lactose, dextrose, sucrose, sorbitol, mannitol, xylitol, erythritol, maltitol, maltodextrin, glycerol, ethanol, or combinations thereof.

In the present disclosure, the pharmaceutical composition may optionally include other pharmaceutically acceptable additives such as excipients, diluents, antioxidants, buffering agents, or preservatives. Additionally, it may further include fillers, bulking agents, humectants, disintegrants, dispersants, surfactants, binders, or lubricants.

Another aspect of the present disclosure provides a quasi-drug including exosomes isolated from induced pluripotent stem cell (iPSC)-derived mesenchymal stem cells as an active ingredient for preventing stretch marks, enhancing skin elasticity, preventing skin aging, or promoting skin regeneration.

The formulation of the quasi-drug composition according to the present disclosure may include, but is not limited to, body cleanser, disinfectant, cleansing agent, kitchen detergent, wet tissue, soap, hand wash, or ointment.

Another aspect of the present disclosure provides a topical composition including exosomes isolated from induced pluripotent stem cell (iPSC)-derived mesenchymal stem cells as an active ingredient for preventing stretch marks, enhancing skin elasticity, preventing skin aging, or promoting skin regeneration.

Another aspect of the present disclosure provides a method for preparing a cosmetic composition for preventing stretch marks, enhancing skin elasticity, preventing skin aging, or promoting skin regeneration, the method including the steps of:
isolating exosomes from stem cells or a culture thereof.

In an embodiment of the present disclosure, the isolation step may comprise isolating exosomes from induced pluripotent stem cell-derived mesenchymal stem cells or from a culture thereof.

In an embodiment of the present disclosure, the stem cell may be an autologous or allogeneic stem cell, may be derived from any type of animal including human and non-human mammals, and may be derived from adult tissue or from embryos. For example, the stem cell may be an embryonic stem cell, adult stem cell, induced pluripotent stem cell (iPSC), mesenchymal stem cell derived from an iPSC, BxC stem cell, or BxC-R11 stem cell, but is not limited thereto.

In the isolation step, the culture medium of the stem cells may be centrifuged at 200-400 ×g for 5 to 20 minutes to remove remaining cells and cell debris. The supernatant is then collected and centrifuged at 9,000-12,000 ×g for 60 to 80 minutes at high speed. After collecting the resulting supernatant again, it may be centrifuged at 90,000-120,000 ×g for 80 to 100 minutes, and the supernatant is discarded to obtain the exosomes remaining as the pellet.

In an embodiment of the present disclosure, the method may further include a pretreatment step of treating the mesenchymal stem cells derived from iPSCs with hyaluronic acid.

In an embodiment of the present disclosure, the method may further include a selective culturing step of isolating and culturing SSEA-4 (-) cells among the cultured induced pluripotent stem cells to differentiate them into BxC stem cells.

In an embodiment of the present disclosure, the method may further include an exosome production step of culturing the stem cells in a cell culture medium.

The exosome production step of the present disclosure refers to the process of inducing the secretion or production of exosomes from stem cells. The cell culture medium used in the present disclosure may include any medium commonly used for culturing stem cells in the art, including but not limited to commercially available or synthetically prepared media such as DMEM (Dulbecco's Modified Eagle's Medium), MEM (Minimal Essential Medium), BME (Basal Medium Eagle), RPMI 1640, DMEM/F-10 (Dulbecco's Modified Eagle's Medium: Nutrient Mixture F-10), DMEM/F-12 (Dulbecco's Modified Eagle's Medium: Nutrient Mixture F-12), α-MEM (α-Minimal Essential Medium), G-MEM (Glasgow's Minimal Essential Medium), IMDM (Iscove's Modified Dulbecco's Medium), KnockOut DMEM, and E8 (Essential 8 Medium).

In an embodiment of the present disclosure, the cell culture medium may further include components such as carbon sources, nitrogen sources, trace elements, amino acids, and antibiotics.

In an embodiment of the present disclosure, the exosome production step may include an additional culture step of culturing stem cells in fetal bovine serum (FBS) from which exosomes have been removed.

The use of exosome-depleted FBS in the cell culture medium prevents contamination from exosomes originating from FBS, which are abundant in regular FBS, thereby ensuring that only exosomes secreted by the stem cells are obtained.

Another aspect of the present disclosure provides a method for preparing a composition containing exosomes for preventing stretch marks, enhancing skin elasticity, preventing skin aging, or promoting skin regeneration, the method including:
a first culturing step of culturing induced pluripotent stem cells (iPSCs) in a medium;
a selective culturing step of isolating SSEA-4 (-) cells among the cultured iPSCs and culturing same to differentiate into BxC stem cells;
a second culturing step of culturing the BxC stem cells to differentiate into mesenchymal stem cells (MSCs);
a pretreatment step of treating the mesenchymal stem cells with hyaluronic acid (HA);
a production step of culturing the pretreated mesenchymal stem cells to produce exosomes; and
an isolation step of isolating exosomes from the mesenchymal stem cells or a culture thereof.

In an embodiment of the present disclosure, the first culturing step may include culturing the iPSCs in a medium containing FBS and bFGF for 1 to 10 days.

In an embodiment of the present disclosure, the selective culturing step may include isolating SSEA-4 (-) cells among the iPSCs and culturing them in a medium containing FBS and bFGF for 1 to 10 days to differentiate into BxC stem cells.

In an embodiment of the present disclosure, the pretreatment step may include culturing the mesenchymal stem cells in a medium containing hyaluronic acid at a concentration of 0.1 to 1000 µg/mL, 0.5 to 1000 µg/mL, 1 to 500 µg/mL, 1 to 200 µg/mL, 1 to 100 µg/mL, 1 to 80 µg/mL, 1 to 60 µg/mL, or 10 to 60 µg/mL, for example, 40 µg/mL.

In an embodiment of the present disclosure, the production step may include an additional culturing step of culturing the mesenchymal stem cells in exosome-depleted fetal bovine serum (FBS).

Another aspect of the present disclosure provides a method for preventing stretch marks, enhancing skin elasticity, preventing skin aging, or promoting skin regeneration, the method including a step of:
contacting the skin of a subject with exosomes isolated from mesenchymal stem cells (MSCs) derived from induced pluripotent stem cells (iPSCs).

In an embodiment of the present disclosure, the exosomes may include exosomes derived from BxC-R11 stem cells (BxC-R11e).

As used herein, the term "administration" refers to providing a predetermined substance to a patient via any suitable method, and the cosmetic or pharmaceutical composition of the present disclosure may be administered orally or non-orally via any route capable of reaching the target tissue. In addition, the composition may also be administered using any device capable of delivering the active ingredient to a target cell.

As used herein, the term "subject" is not particularly limited and may include, for example, humans, monkeys, cattle, horses, sheep, pigs, chickens, turkeys, quails, cats, dogs, mice, rats, rabbits, or guinea pigs, and preferably, may be a human, but is not limited thereto.

In an embodiment of the present disclosure, the pharmaceutical composition may be administered alone or, more typically, administered in combination with a pharmaceutically acceptable carrier selected in consideration of the route of administration and standard pharmaceutical practice.

Another aspect of the present disclosure provides the use of exosomes isolated from induced pluripotent stem cell (iPSC)-derived mesenchymal stem cells for preventing stretch marks, enhancing skin elasticity, preventing skin aging, or promoting skin regeneration.

### Advantageous Effects of Invention

The present disclosure relates to a composition for inhibiting skin aging, including stem cell-derived exosomes and a method for preparing the same. The composition including exosomes according to the present disclosure exhibits excellent effects in preventing stretch marks, enhancing skin elasticity, preventing skin aging, or promoting skin regeneration, and thus can be utilized in various cosmetic and pharmaceutical compositions.

### Brief Description of Drawings

FIG. 1 is a graph showing the degree of improvement in cell proliferation in aged human dermal fibroblasts (aged HDFs) treated with the control (vehicle and hyaluronic acid) and the test group [R11e (HA-iMSC-EVs) exosomes] according to an embodiment of the present disclosure.
FIG. 2 is a graph showing the level of NANOG gene expression in aged human dermal fibroblasts (aged HDFs) treated with the control [vehicle, hyaluronic acid, and young human dermal fibroblasts] and the test group [R11e (HA-iMSC-EVs) exosomes] according to an embodiment of the present disclosure.

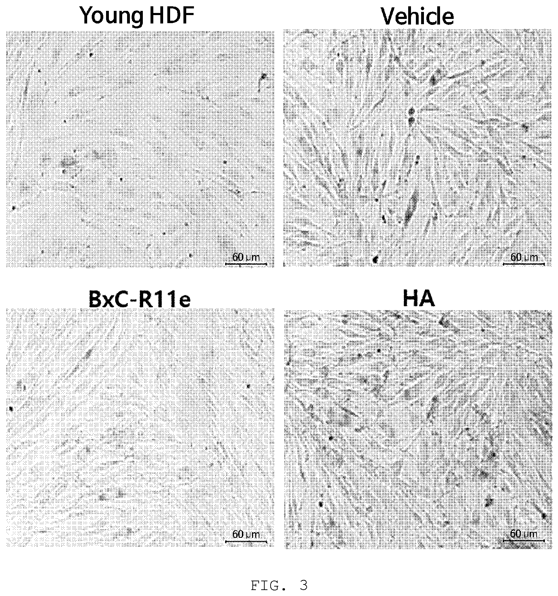

FIG. 3 is a photograph showing the degree of SA-β-Gal (senescence-associated beta-galactosidase) activity in aged human dermal fibroblasts (aged HDFs) treated with the control [vehicle, hyaluronic acid, and young human dermal fibroblasts] and the test group [R11e (HA-iMSC-EVs) exosomes] according to an embodiment of the present disclosure.

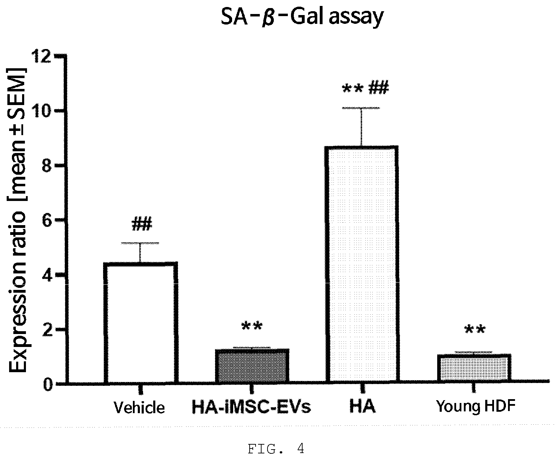

FIG. 4 is a diagram showing the results of SA-β-Gal (senescence-associated beta-galactosidase) activity in aged human dermal fibroblasts (aged HDFs) treated with the control [vehicle, hyaluronic acid, and young human dermal fibroblasts] and the test group [R11e (HA-iMSC-EVs) exosomes] according to an embodiment of the present disclosure.
FIG. 5 is a graph showing the level of EGF (epidermal growth factor) gene expression in skin fibroblasts treated with the control [vehicle, hyaluronic acid, and young human dermal fibroblasts] and the test group [R11e (HA-iMSC-EVs) exosomes] according to an embodiment of the present disclosure.

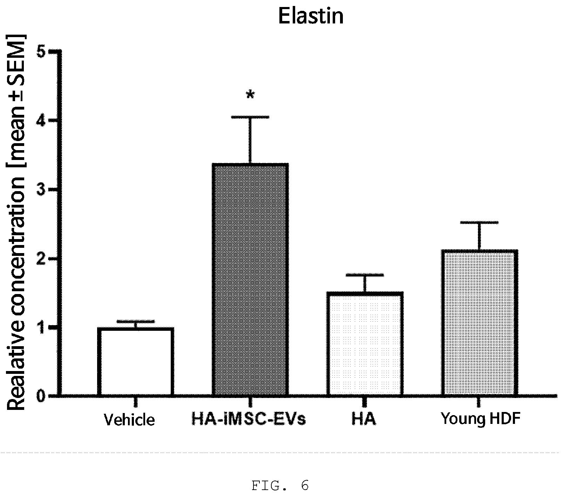

FIG. 6 is a graph showing the level of elastin gene expression in skin fibroblasts treated with the control [vehicle, hyaluronic acid, and young human dermal fibroblasts] and the test group [R11e (HA-iMSC-EVs) exosomes] according to an embodiment of the present disclosure.

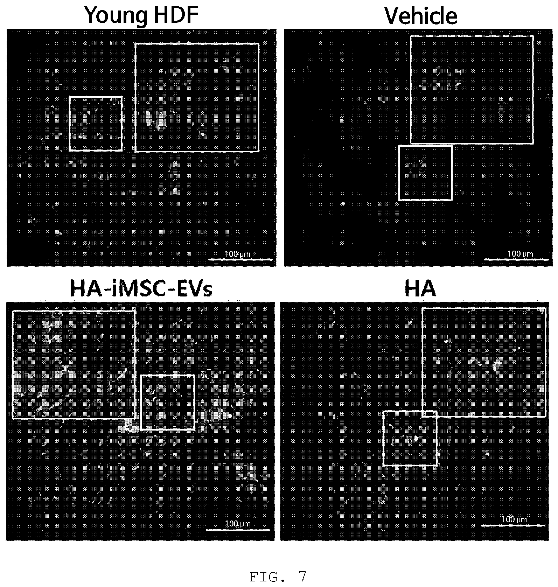

FIG. 7 is a photograph showing the expression of elastin protein in skin fibroblasts treated with the control [vehicle, hyaluronic acid, and young human dermal fibroblasts] and the test group [R11e (HA-iMSC-EVs) exosomes] according to an embodiment of the present disclosure.

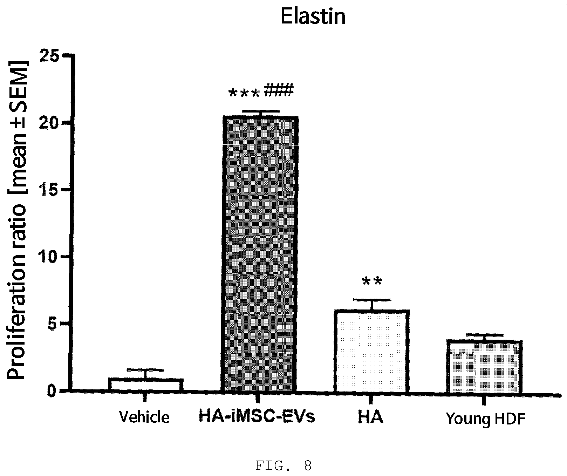

FIG. 8 is a graph showing the expression of elastin protein in skin fibroblasts treated with the control [vehicle, hyaluronic acid, and young human dermal fibroblasts] and the test group [R11e (HA-iMSC-EVs) exosomes] according to an embodiment of the present disclosure.
FIG. 9 is a graph showing the collagen expression levels measured after treating fibroblasts with dexamethasone according to an embodiment of the present disclosure.

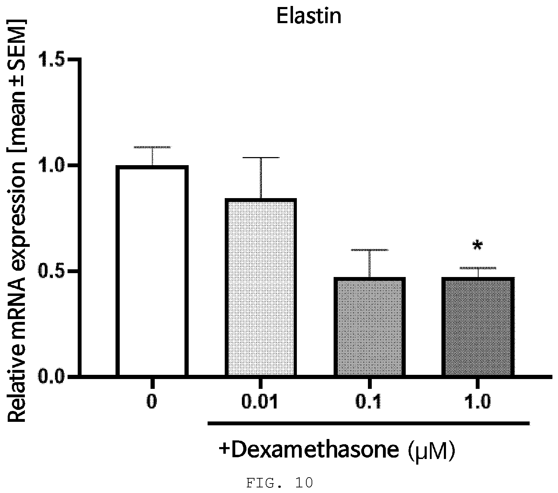

FIG. 10 is a graph showing the elastin expression levels measured after treating fibroblasts with dexamethasone according to an embodiment of the present disclosure.

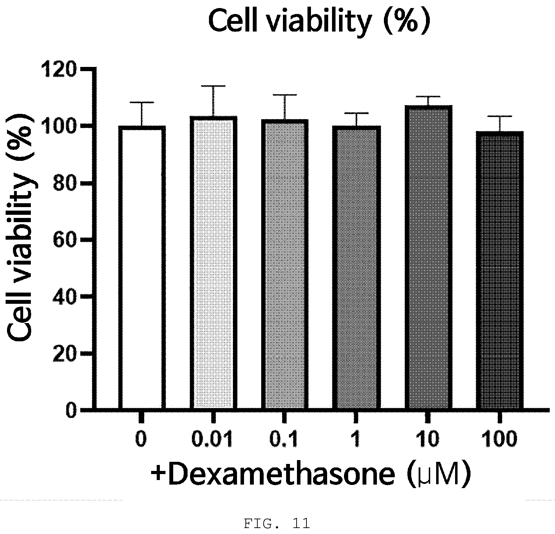

FIG. 11 is a graph showing the results of measuring cell viability after treating fibroblasts with dexamethasone according to an embodiment of the present disclosure.

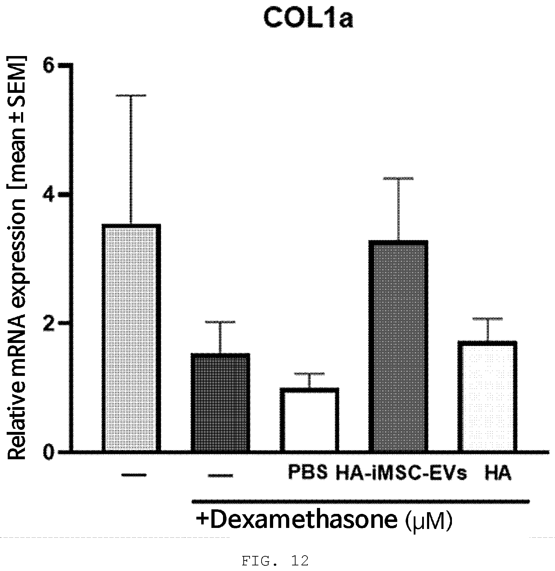

FIG. 12 is a graph showing the expression of collagen (COLla) genes in skin fibroblasts treated with the control [fibroblasts cultured in serum-free DMEM], negative control (vehicle/PBS), positive control (100 µg/mL hyaluronic acid), and the test substance [R11e (HA-iMSC-EVs) exosomes] according to an embodiment of the present disclosure.

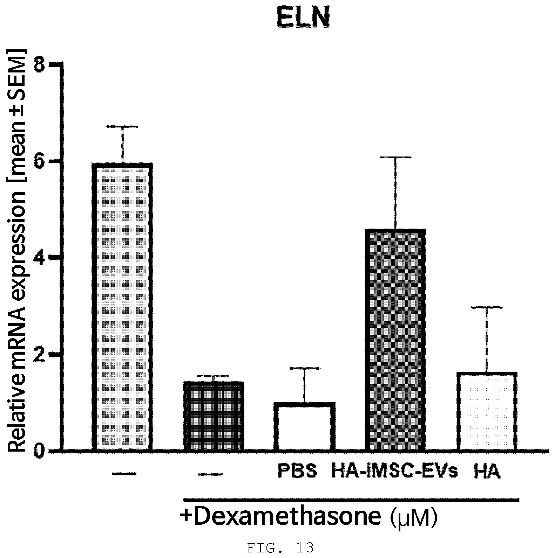

FIG. 13 is a graph showing the expression of elastin (ELN) genes in skin fibroblasts treated with the control [fibroblasts cultured in serum-free DMEM], negative control (vehicle/PBS), positive control (100 µg/mL hyaluronic acid), and the test substance [R11e (HA-iMSC-EVs) exosomes] according to an embodiment of the present disclosure.

### Best Mode for Carrying out the Invention

A cosmetic composition for preventing stretch marks, enhancing skin elasticity, preventing skin aging, or promoting skin regeneration, the composition including as an active ingredient, exosomes isolated from mesenchymal stem cells (MSCs) derived from induced pluripotent stem cells (iPSCs).

### Mode for Carrying Out the Invention

Hereinafter, a better understanding of the present disclosure may be obtained through the following examples, which are set forth to illustrate, but are not to be construed to limit, the scope of the present disclosure.

### EXAMPLE 1: Culture of Induced Pluripotent Stem Cell-Derived Mesenchymal Stem Cells

Induced pluripotent stem cells (iPSCs) were cultured for 7 days in DMEM supplemented with 10% fetal bovine serum (FBS) and 10 ng/mL of bFGF. Thereafter, SSEA-4 (stage-specific embryonic antigen 4)-negative cells, which do not express SSEA-4 protein on the cell surface, were isolated from the cultured iPSCs by FACS analysis to obtain precursor cells of mesenchymal stem cells derived from iPSCs. The isolated SSEA-4 (-) cells were subcultured for an additional 7 days in DMEM supplemented with 10% FBS and 10 ng/mL bFGF to produce BxC stem cells.

The BxC stem cells were then further cultured in a culture medium comprising high-glucose DMEM (Gibco, USA), 10% FBS (HyClone, USA), and 1% MEM Non-Essential Amino Acids Solution (100X, Gibco, USA) to fully differentiate them into induced pluripotent stem cell-derived mesenchymal stem cells.

### EXAMPLE 2: Isolation of Hyaluronic Acid-Pretreated Exosomes (BxC-R11e)

The mesenchymal stem cells derived from iPSCs obtained in Example 1 were cultured for 24 hours in a high-glucose DMEM medium supplemented with 10% FBS, 1% MEM Non-Essential Amino Acids Solution, and 40 µg/mL of hyaluronic acid to produce hyaluronic acid-pretreated mesenchymal stem cells (BxC-R11 stem cells).

After the culture was completed, the BxC-R11 stem cells were washed and further cultured for 72 hours in a medium supplemented with 10% exosome-depleted FBS.

After 72 hours, the culture medium was collected and centrifuged at 300 ×g for 10 minutes to remove remaining cells and debris. The supernatant was collected and filtered through a 0.22 µm filter, followed by highspeed centrifugation at 10,000 ×g and 4°C for 70 minutes. The resulting supernatant was further centrifuged using an ultracentrifuge at 100,000 ×g and 4°C for 90 minutes. After discarding the supernatant, the pellet containing the exosomes was resuspended in PBS to isolate hyaluronic acid-pretreated exosomes (hereinafter referred to as BxC-R11e exosomes), which were used in the experiments described below.

### EXAMPLE 3: Confirmation of Improved Cell Proliferation in Aged Fibroblasts

To confirm whether the BxC-R11e exosomes prepared in Example 2 improve the reduced cell proliferation in aged fibroblasts (human dermal fibroblasts, HDFs), the exosomes were applied and cell proliferation was compared after 24 hours.

Aged fibroblasts (Aged HDFs) were seeded in 96-well plates at a density of 5,000 cells per well and cultured for at least 16 hours at 37°C and 5% CO₂ in basic medium (high-glucose DMEM, 10% FBS, 100 U/mL penicillin-streptomycin) for cell attachment. The treatment groups were as follows: test groups were treated with BxC-R11e exosomes (1-100 µg/mL) in serum-free DMEM; the negative control group was treated with PBS; and the positive control group was treated with 100 µg/mL hyaluronic acid (HA). All groups were incubated for 24 hours.

After 24 hours, cell proliferation was measured using the Cell Count Kit-8 (CCK-8) assay (Enzo, New York, NY, USA). CCK-8 solution was added and incubated for 2 hours at 37°C, after which optical density (OD) was measured at 450 nm using a microplate reader. The values for each well (n = 3 per group) were averaged and the results were expressed relative to the mean value of the negative control (vehicle), as shown in FIG. 1 and Table 1.

**TABLE 1**

| | Vehicle | HA-iMSC-EVs (R11e) | | | HA |
|---|---|---|---|---|---|
| | | 1 *µ*g/mL | 10 *µ*g/mL | 100 *µ*g/mL | |
| Cell proliferation | 1 | 1.18 | 1.26 | 1.39 | 1. 18 |

As shown in FIG. 1 and Table 1, after 24 hours of treatment, the cell proliferation value of the negative control group (PBS) was set as 1. The positive control group treated with HA showed an 18% increase (1.18). The group treated with 1 µg/mL of R11e showed an 18% increase in proliferation compared to the negative control, the group treated with 10 µg/mL R11e showed a 26% increase, and the group treated with 100 µg/mL R11e showed a 39% increase, confirming that the R11e exosomes significantly enhanced cell proliferation compared to both the negative and positive control groups.

### EXAMPLE 4: Confirmation of Cell Regeneration Effect

To confirm the regeneration (renewal) effect on fibroblasts, human dermal fibroblasts were treated with the hyaluronic acid-pretreated exosomes (BxC-R11e) produced in Example 2, and the expression of the NANOG gene was measured after 24 hours.

Aged human dermal fibroblasts (Aged HDFs) were seeded at 20,000 cells per well in 12-well plates and cultured for at least 16 hours at 37°C in 5% CO₂ using basic medium (high-glucose DMEM, 10% FBS, 100 U/mL penicillin-streptomycin) for cell attachment. The test group was treated with BxC-R11e (1-100 µg/mL) in serum-free DMEM; the negative control group was treated with PBS; and the positive control group was treated with 100 µg/mL hyaluronic acid (HA), followed by incubation for 24 hours.

After 24 hours, total RNA was extracted using Trizol (Invitrogen), and mRNA expression of the target genes was measured from an equal amount of total RNA using real-time PCR. Each expression value was normalized to GAPDH expression using the 2^{-ΔΔCt} method, and the results are shown in FIG. 2 and Table 2.

**TABLE 2**

| | Vehicle | HA-iMSC-EVs (BxC-R11e) | | | HA | Young HDFs |
|---|---|---|---|---|---|---|
| | | 1 *µ*g/mL | 10 *µ*g/mL | 100 *µ*g/mL | | |
| NANOG Expression | 1 | 0.50 | 1.17 | 3.70 | 1.01 | 3.97 |

As shown in FIG. 2 and Table 2, the expression of the NANOG gene was measured 24 hours after treatment, using the PBS-treated group as the negative control (expression set to 1). The positive control group treated with HA showed a similar level (1.01), indicating no significant difference from the negative control. In contrast, the group treated with 10 µg/mL of R11e showed a 17% increase, and the group treated with 100 µg/mL R11e showed a 370% increase in NANOG expression, demonstrating a level similar to that of non-aged young HDFs.

### EXAMPLE 5: Confirmation of Decrease in SA-β-Gal Activity in Aged Cells

To confirm whether the increased SA-β-Gal (senescence-associated beta-galactosidase) activity in aged cells could be reduced, skin fibroblasts were treated with BxC-R11e exosomes produced in Example 2, and cell proliferation was measured after 24 hours.

Aged human dermal fibroblasts (Aged HDFs) were seeded at 20,000 cells per well in 12-well plates and cultured for at least 16 hours at 37°C in 5% CO₂ using basic medium (high-glucose DMEM, 10% FBS, 100 U/mL penicillin-streptomycin) for cell attachment. The test group was treated with 100 µg/mL BxC-R11e in serum-free DMEM; the negative control group was treated with PBS; and the positive control group was treated with 100 µg/mL hyaluronic acid (HA), followed by incubation for 24 hours.

SA-β-Gal assay was conducted using the Senescence Detection Kit (Abcam, Cambridge, UK). After 24 hours of incubation, cells were fixed with 4% paraformaldehyde at room temperature for 20 minutes. SA-β-Gal staining was performed following the manufacturer's protocol and observed under a microscope (see FIG. 3). Senescent cells were stained blue, and the stained area was quantified using the ImageJ program. The results are shown in FIG. 4 and Table 3.

**TABLE 3**

| | Vehicle | HA-iMSC-EVs (BxC-R11e) | HA | Young HDFs |
|---|---|---|---|---|
| SA- β -Gal Activity | 4. 45 | 1.25 | 8. 67 | 1 |

As shown in FIG. 4 and Table 3, 24 hours after treatment, the negative control group exhibited SA-β-Gal activity approximately four times higher (4.45) than the non-aged Young HDFs. In contrast, the group treated with R11e showed an activity level of 1.25, representing a 3.6-fold reduction compared to the negative control, and a level comparable to that of the Young HDF group.

### EXAMPLE 6: Increase of Epidermal Growth Factor (EFG) and Elastin Gene Expression

To verify the regeneration (renewal) effect of the test substance on fibroblasts, human dermal fibroblasts were treated with hyaluronic acid-pretreated exosomes (BxC-R11e) produced in Example 2, and the expression levels of the EGF (epidermal growth factor) and elastin genes were compared after 24 hours.

Aged human dermal fibroblasts (Aged HDFs) were seeded at a density of 20,000 cells/well in 12-well plates and cultured for at least 16 hours at 37°C and 5% CO₂ in basic medium (high-glucose DMEM, 10% FBS, 100 U/mL penicillin-streptomycin) for cell attachment. The test group was treated with 100 µg/mL BxC-R11e in serum-free DMEM; the negative control group was treated with PBS; and the positive control group was treated with 100 µg/mL hyaluronic acid (HA). All groups were cultured for 24 hours.

After 24 hours, total RNA was extracted using Trizol (Invitrogen), and mRNA expression of each gene was quantified from equal amounts of total RNA using real-time PCR. Each value was normalized against GAPDH expression using the 2^{-ΔΔCt} method. The results are shown in FIGS. 5 and 6 and Table 4.

**TABLE 4**

| | Vehicle | HA-iMSC-EVs (BxC-R11e) | HA | Young HDFs |
|---|---|---|---|---|
| EGF Expression | 1 | 2. 65 | 0.67 | 2.45 |
| Elastin Expression | 1 | 3.39 | 1.51 | 2.14 |

As shown in FIGS. 5 and 6 and Table 4, gene expression measured 24 hours after treatment indicated that EGF expression increased by 2.7-fold and elastin expression increased by 3.4-fold in the BxC-R11e-treated test group compared to the negative control group (PBS-treated).

### EXAMPLE 7: Elastin Protein Expression

Aged human dermal fibroblasts (Aged HDFs) were seeded at a density of 40,000 cells/well in 12-well plates and cultured for at least 24 hours at 37°C and 5% CO₂ in basic medium (high-glucose DMEM, 10% FBS, 100 U/mL penicillin-streptomycin) for cell attachment. The test group was treated with 100 µg/mL BxC-R11e in DMEM containing 5% FBS (serum-free DMEM); the negative control group was treated with PBS; and the positive control group was treated with 100 µg/mL hyaluronic acid (HA). All groups were cultured for 48 hours.

After 48 hours, the cells were fixed with 100% methanol, washed with PBS, and incubated with a primary antibody (anti-tropoelastin antibody, Elastin Products, Owensville, MI). After washing, the cells were incubated with a FITC-conjugated secondary antibody and observed under a fluorescence microscope (see FIG. 7). The stained area was quantified using ImageJ software, and the results are shown in FIG. 8 and Table 5.

**TABLE 5**

| | Vehicle | HA-iMSC-EVs (BxC-R11e) | HA | Young HDFs |
|---|---|---|---|---|
| EGF protein expression | 1 | 2. 65 | 0. 67 | 2.45 |

Generally, in addition to the increased expression of elastin protein, functional performance requires the assembly of elastin into elastic fibers. In *in vitro* environments, the assembly of elastin protein into elastic fibers takes time.

Accordingly, as confirmed in FIGS. 7 and 8 and Table 4, elastin protein expression increased in Young HDFs, but no elastic fibers were observed. In contrast, in the BxC-R11e-treated group, not only was elastin protein expression increased, but elastic fiber formation was also observed. Thus, the hyaluronic acid-pretreated exosomes (BxC-R11e) were confirmed to accelerate the assembly of elastic fibers.

### EXAMPLE 8: Prevention Effect Against Stretch Mark

### 8-1. Suppression of Collagen and Elastin Expression by Dexamethasone Treatment

Human dermal fibroblasts (HDFs) were seeded at a density of 20,000 cells/well in 12-well plates and cultured for over 24 hours at 37°C, 5% CO₂ in basic medium (high-glucose DMEM, 10% FBS, 100 U/mL penicillin-streptomycin) to allow cell attachment.

Afterward, dexamethasone, a type of corticosteroid drug, was added to serum-free DMEM at various concentrations and applied to the fibroblasts. After 24 hours of treatment, the fibroblasts were harvested, and the gene expression levels of collagen and elastin were analyzed. The results are shown in FIGS. 9 to 11.

As shown in FIGS. 9 and 10, dexamethasone treatment suppressed the expression of both collagen and elastin. Meanwhile, as shown in FIG. 11, dexamethasone treatment did not affect cell viability, indicating that the concentrations used were not cytotoxic to the fibroblasts.

### 8-2. Stretch Mark Prevention Effect by Treatment with Hyaluronic Acid-Pretreated Exosomes

To confirm the stretch mark prevention effect, gene expression changes in collagen and elastin were measured 24 hours after applying the test substances to fibroblasts treated with the glucocorticoid steroid dexamethasone.

Human dermal fibroblasts (HDFs) were seeded at a density of 20,000 cells/well in 12-well plates and cultured for at least 16 hours at 37°C, 5% CO₂ in basic medium (high-glucose DMEM, 10% FBS, 100 U/mL penicillin-streptomycin) for cell attachment. After mixing dexamethasone into serum-free DMEM, the test substance or control substances were also added and applied to the cells, followed by 24 hours of incubation. The experimental groups were as follows: the normal control group consisted of fibroblasts cultured in serum-free DMEM only; the negative control group was treated with vehicle (PBS); the positive control group was treated with 100 µg/mL of hyaluronic acid (HA); and the test group was treated with 100 µg/mL of hyaluronic acid-pretreated exosomes (HA-iMSC-EVs, BxC-R11e).

After 24 hours, cells were harvested, and total RNA was extracted using Trizol (Invitrogen). Real-time PCR analysis was performed using equal amounts of total RNA, and gene expression levels were normalized to GAPDH expression using the 2^{-ΔΔCt} method.

**[TABLE 6]**

| Gene | Normal control | Dexamethasone only | Dexamethasone +PBS | Dexamethasone +R11e | Dexamethasone +HA |
|---|---|---|---|---|---|
| COLla | 3.55 | 1.53 | 1.00 | 3.30 | 1.73 |
| ELN | 5.96 | 1.44 | 1.00 | 4.59 | 1.65 |

As shown in FIGS. 12 and 13 and Table 6, treatment with dexamethasone alone led to a 57% and 75% decrease in COLla and ELN gene expression, respectively, compared to the normal control group. In contrast, after 24 hours of treatment, the positive control group (HA-treated) showed increases in COLla and ELN expression of approximately 73% (1.73) and 65% (1.65), respectively, compared to the PBS group, which was the negative control group the cell number of which was set to be 1. In the test group treated with hyaluronic acid-pretreated exosomes (HA-iMSC-EVs, BxC-R11e), COLla expression increased by 330% and ELN expression increased by 459% compared to the vehicle group.

Clinically, stretch mark (striae distensae) lesions are characterized by decreased expression of collagen and elastin. Currently, treatment methods generally include drugs and laser therapies aimed at increasing collagen in the dermis. Therefore, the hyaluronic acid-pretreated exosomes (HA-iMSC-EVs, BxC-R11e) of the present disclosure were confirmed to significantly increase collagen and elastin gene expression in a dexamethasone-treated fibroblast model, thereby demonstrating effectiveness in preventing stretch marks.

### Industrial Applicability

The present disclosure relates to a composition for inhibiting skin aging including stem cell-derived exosomes and a method for preparing the same. More specifically, it relates to a composition including exosomes isolated from mesenchymal stem cells or a culture thereof, which exhibit excellent effects in preventing stretch marks, enhancing skin elasticity, preventing skin aging, or promoting skin regeneration.

## Claims

1. A cosmetic composition for preventing stretch marks, enhancing skin elasticity, preventing skin aging, or promoting skin regeneration, comprising exosomes isolated from induced pluripotent stem cell (iPSC)-derived mesenchymal stem cells (MSCs) as an active ingredient.

2. The cosmetic composition of claim 1, wherein the induced pluripotent stem cell (iPSC)-derived mesenchymal stem cells are differentiated from precursor cells that do not express the stage-specific embryonic antigen 4 (SSEA-4) protein.

3. The cosmetic composition of claim 1, wherein the induced pluripotent stem cells are human-derived induced pluripotent stem cells.

4. The cosmetic composition of claim 1, wherein the induced pluripotent stem cell-derived mesenchymal stem cells are pretreated with a pretreatment substance.

5. The cosmetic composition of claim 4, wherein the pretreatment substance is hyaluronic acid.

6. The cosmetic composition of claim 1, wherein the composition is in a formulation form selected the group consisting of: solution, topical ointment, cream, foam, nourishing toner, softening toner, pack, softener, emulsion, makeup base, essence, soap, liquid cleanser, bath preparation, sunscreen cream, sun oil, suspension, emulsion, paste, gel, lotion, powder, soap, surfactant-containing cleansing agent, oil, powder foundation, emulsion foundation, wax foundation, patch, and spray.

7. A quasi-drug for preventing stretch marks, enhancing skin elasticity, preventing skin aging, or promoting skin regeneration, comprising exosomes isolated from induced pluripotent stem cell (iPSC)-derived mesenchymal stem cells (MSCs) as an active ingredient.

8. The quasi-drug of claim 7, wherein the induced pluripotent stem cell-derived mesenchymal stem cells are pretreated with a pretreatment substance.

9. The quasi-drug of claim 8, wherein the pretreatment substance is hyaluronic acid.

10. A quasi-drug for preventing stretch marks, enhancing skin elasticity, preventing skin aging, or promoting skin regeneration, comprising exosomes isolated from induced pluripotent stem cell (iPSC)-derived mesenchymal stem cells (MSCs) as an active ingredient.

11. The external skin composition of claim 10, wherein the mesenchymal stem cells derived from the iPSCs are pretreated with a pretreatment substance and are formulated as a topical composition.

12. The external skin composition of claim 8, wherein the pretreatment substance is hyaluronic acid.
